# EUROPEAN PATENT SPECIFICATION

(11) **EP 3 167 815 B1**
(45) Date of publication and mention of the grant of the patent: **17.02.2021**
(21) Application number: 16198333.3
(22) Date of filing: 11.11.2016
(51) Int. Cl.: A61B 17/064

(54) **EXPANDING HERNIA FIXATION TACK**
AUFWEITBARER HERNIENFIXATIONSSCHRAUBE
VIS DE FIXATION EXPANSIBLE POUR HERNIE

(30) Priority: 12.11.2015 US 201514939271
(43) Date of publication of application: 17.05.2017
(73) Proprietor: Covidien LP, Mansfield, MA 02048 (US)
(72) Inventor: RUSSO, Mark, Plantsville, CT Connecticut 06479 (US)
(74) Representative: Soames, Candida Jane

(56) References cited:
- WO-A1-2005/004727
- US-A1- 2006 100 629
- US-A1- 2015 032 130

## Description

### CROSS REFERENCE TO RELATED APPLICATIONS

This application is a continuation-in-part of U.S. Patent Application Serial No. 14/286,142, filed May 23, 2014, which claims the benefit of U.S. Provisional Patent Application No. 61/857,709, filed July 24, 2013.

### TECHNICAL FIELD

The present disclosure relates to surgical devices, systems, and/or methods for performing surgical procedures. More specifically, the present disclosure relates to fasteners for surgical fastener applying devices and/or systems for performing minimally invasive surgical procedures, and methods of use thereof.

### BACKGROUND

Various surgical procedures require devices capable of applying fasteners to tissue to form tissue connections or to secure objects to tissue. For example, during hernia repair it is often desirable to fasten a mesh to body tissue. In certain hernias, such as direct or indirect inguinal hernias, a part of the intestine protrudes through a defect in the abdominal wall to form a hernial sac. The defect may be repaired using an open surgery procedure in which a relatively large incision is made and the hernia is closed outside the abdominal wall by suturing. The mesh is attached with sutures over the opening in the abdominal wall to provide reinforcement.

Minimally invasive, *e.g.,* endoscopic or laparoscopic, surgical procedures are currently available to repair a hernia. In laparoscopic procedures, surgery is performed in the abdomen through a small incision while in endoscopic procedures, surgery is performed through narrow endoscopic tubes or cannulas inserted through small incisions in the body. Laparoscopic and endoscopic procedures generally utilize long and narrow devices capable of reaching remote regions within the body and are configured to form a seal with the incision or tube they are inserted through. Additionally, these devices are typically capable of being actuated remotely, that is, from outside the body.

Currently, minimally invasive surgical techniques for hernia repair utilize surgical fasteners, e.g., surgical tacks, staples, and clips, to secure the mesh to the tissue to provide reinforcement and structure for encouraging tissue ingrowth. Surgical fasteners are often applied through an elongated device for delivery to the mesh, and are manipulated from outside a body cavity. While diameters of these elongated devices can vary in size, elongated devices with smaller diameters enable access through smaller incisions or openings, and thereby limit tissue damage.

There is a growing trend to utilize hernia meshes with large pore sizes. Hernia meshes with large pore sizes are often positioned to cover larger areas in order to limit mesh migration. Thus, fixation of large pore hernia meshes may require utilizing minimum quantities of larger surgical fasteners of larger diameter elongated devices and/or utilizing maximum quantities of small surgical fasteners of smaller diameter elongated devices.

Accordingly, a need exists to provide fasteners that can be delivered through small diameter elongated devices and that are capable of effectively securing hernia meshes having large pore sizes utilizing only minimum quantities of such fasteners

US2015/032130 relates to a surgical fastener which includes a head section and a tissue snaring section. The head section includes an engagement member pivotably disposed on an outer surface of the head section. The tissue snaring section defines a longitudinal axis and extends away from the head section. At least one helical thread defined along an outer surface of the tissue snaring section. The tissue snaring section is configured to rotate about the longitudinal axis in a first radial direction, and the engagement member is configured pivot outwardly from the head section in the first radial direction.

US 2006/100629 relates to a method and apparatus for repair of torn rotator cuff tendons. A cannula and an orthopedic fastener are passed over a guide wire to the site of the tear through a minimal incision. The fastener includes a plurality of pivotally mounted vanes which are compressible in the cannula and extendable at the repair site for securing a torn tendon to the bone.

WO 2005/004727 relates to surgical fasteners and to surgical fastening devices.

### SUMMARY

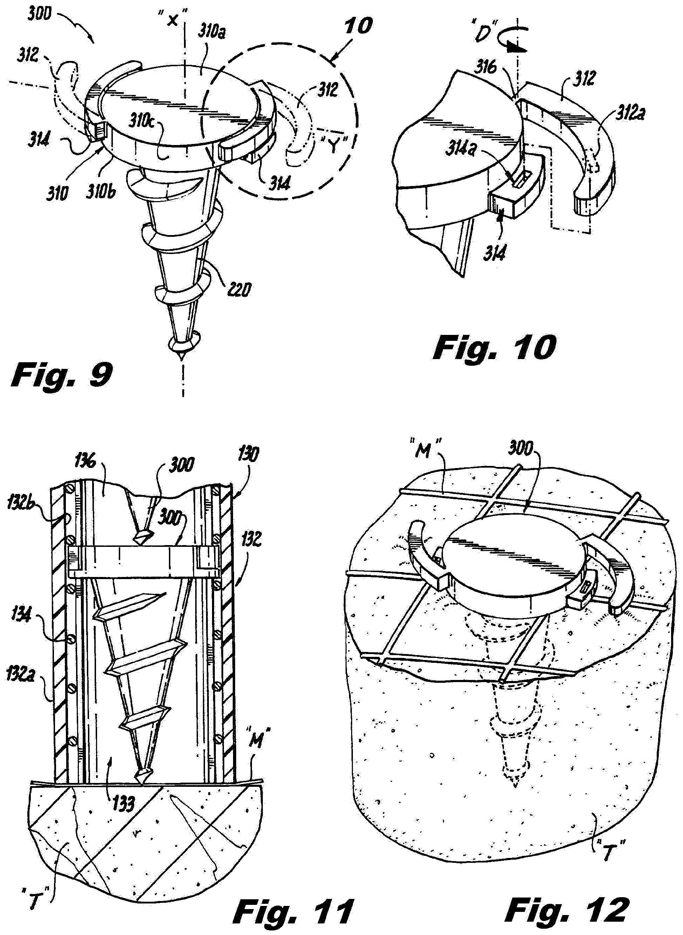

The present invention is defined by the features of the independent claim. Preferred embodiments are given in the dependent claims. A fastener according to the invention is described in figures 9-12 and corresponding passages in the description.

Accordingly, the present disclosure is directed to hernia fixation tacks/fasteners that can be delivered through small elongated devices (*e.g.,* 5mm diameter) and that are expandable to cover larger areas of hernia meshes (*e.g.,* mesh areas larger than 5mm). The presently described fasteners have a head and a shank. The head includes hinged wings that move between a constrained state and an extended state. In the constrained state, the hinged wings are disposed in close approximation to the head to enable the fastener to be advanced through small elongated devices such as 5mm diameter cannulas or delivery devices. The hinged wings expand outwardly from the head in the extended state when deployed from a delivery device. In the extended state, the hinged wings project outwardly beyond areas larger than the diameter of the delivery device. Advantageously, the presently described fasteners can be advanced through small and or minimally invasive passages and deployed in vivo to enable securement of large pore meshes (*e.g*., meshes with pores larger than the diameter of the delivery device) to a target tissue site.

In one aspect of the present disclosure, a fastener for securement to a target tissue site includes a head, a shank extending from the head, and one or more hinged wings secured to the head. The shank includes a thread that extends along an outer surface of the shank. The thread is configured to secure the fastener to the target tissue site.

The hinged wing includes a shoulder and is movable relative to the head between a constrained state and an extended state. The shoulder is engagable with a drive member of a delivery device while the hinged wing is disposed in the constrained state to enable the drive member to translate and rotate the fastener through the delivery device in response to movement of the drive member. The hinged wing is movable toward the extended state in response to deployment of the fastener from the delivery device. In embodiments, the hinged wing may include a living hinge configured to bias the hinged wing toward the extended state. The hinged wing may be a first hinged wing.

The thread of the shank may extend from the outer surface of the shank and around the outer surface of the shank. The thread may define a spiral around the outer surface of the shank along a length of the shank.

In embodiments, the hinged wing may have an arcuate profile that conforms to an outer surface of the head while the hinged wing is disposed in the constrained state.

In some embodiments, the hinged wing may pivot radially outward from the head in a lateral direction as the hinged wing moves toward the extended state.

According to the invention the head supports a ledge that defines a pocket therein and the hinged wing includes a boss extending therefrom. The pocket of the ledge is configured to removably receive the boss of the hinged wing to maintain the hinged wing in the constrained state.

In embodiments, a second hinged wing is secured to the head and may include a second shoulder. The second hinged wing may be movable relative to head between a constrained state and an extended state. The hinged wings may be supported on the head in complementary relation to one another.

In some embodiments, the fastener may be adapted to advance through a 5mm diameter passage of the delivery device while the hinged wing is disposed in the constrained state.

In certain embodiments, the shank may define a longitudinal axis and the hinged wing may define a longitudinal wing axis. The longitudinal wing axis may be disposed in parallel relation to the longitudinal axis of the shank while the hinged wing is disposed in the constrained state. The longitudinal wing axis may be disposed in transverse relation to the longitudinal axis of the shank while the hinged wing is disposed in the extended state.

The hinged wing may define one or more channels in an outer surface thereof. The channel may defines a portion of a helical thread and may be adapted to be engaged by a thread of the delivery device to enable the fastener to advance along the delivery device.

According to another aspect of the present disclosure, a method for securing a mesh to a target tissue site with a fastener is provided. The method includes driving a shoulder of a hinged wing of the fastener to advance the fastener through a delivery device while maintaining the hinged wing of the fastener in a constrained state with respect to a head of the fastener, deploying the head of the fastener from the delivery device to enable the hinged wing of the fastener to move from the constrained state to an extended state in which the hinged wing extends from the head of the fastener, and positioning the hinged wing in contact with the mesh to support the mesh against the target tissue site.

The method may include pivoting the hinged wing radially outward from the head, in a lateral direction, to move the fastener from the constrained state to the extended state.

The method may involve positioning the hinged wing in parallel relation to a shank of the fastener while the hinged wing is disposed in the constrained state. The method may include positioning the hinged wing in transverse relation to the shank of the fastener while the hinged wing is disposed in the extended state.

The method may include securing the shank of the fastener to the target tissue site while the head of the fastener is disposed within the delivery device to apply a tensile force to the hinged wing and move the hinged wing from the constrained state to the extended state as the head of the fastener is deployed from the delivery device.

Advancing of the fastener may include advancing the fastener through a 5mm diameter passage defined in the delivery device. The method may include extending the hinged wing from the head of the fastener to enable the hinged wing to extend beyond a 5mm diameter area at the target tissue site.

According to yet another aspect of the present disclosure, an endoscopic surgical system includes an end effector supporting a drive member and a fastener supported in the end effector. The fastener includes a head, a shank extending from the head, and a hinged wing secured to the head. The shank includes a thread that extends along an outer surface of the shank. The thread is configured to secure the fastener to the target tissue site. The hinged wing includes a shoulder. The hinged wing is movable relative to head between a constrained state and an extended state. The shoulder is engagable with the drive member of the end effector while the hinged wing is disposed in the constrained state to enable the drive member to translate and rotate the fastener through the end effector in response to movement of the drive member. The hinged wing is movable toward the extended state in response to deployment of the fastener from the end effector.

Other aspects, features, and advantages will be apparent from the description, the drawings, and the claims that follow.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description given below, serve to explain the principles of the disclosure, wherein:
Fig. 1 is a perspective view of an endoscopic surgical system in accordance with the present disclosure;
Fig. 2 is a side, cross-sectional view of a loading unit of the endoscopic surgical system shown in Fig. 1, as taken through section line 2-2 of Fig. 1;
Fig. 3 is a perspective view of a portion of the loading unit of Fig. 2 with an outer tube and a coil thereof removed for clarity;
Fig. 4 is a perspective view, with parts separated, of the portion of the loading unit shown in Fig. 3;
Fig. 5A is a perspective view of one embodiment of a fastener in accordance with the present disclosure, the fastener including hinged wings shown in a constrained state;
Fig. 5B is an enlarged, perspective view of one of the hinged wings of the fastener of Fig. 5A shown in an extended state;
Fig. 6 is a top view of the fastener of Fig. 5A with the hinged wings thereof shown in the extended state, the hinged wings thereof also shown in phantom to illustrate the hinged wings in the constrained state;
Figs. 7 and 8 are progressive views showing the endoscopic surgical system of Fig. 1 securing a mesh to a target tissue site with the fastener of Fig. 5A;
Fig. 9 is a perspective view of another embodiment of a fastener in accordance with the present disclosure;
Fig. 10 is an enlarged view of the indicated area of detail shown in Fig. 9;
Figs. 11 and 12 are progressive views showing the endoscopic surgical system of Fig. 1 securing a mesh to a target tissue site with the fastener of Fig. 9;
Fig. 13 is a perspective view of yet another embodiment of a fastener in accordance with the present disclosure;
Fig. 14 is an enlarged view of the indicated area of detail shown in Fig. 13;
Figs. 15 to 17 are progressive views showing the endoscopic surgical system of Fig. 1 securing a mesh to a target tissue site with the fastener of Fig. 13; and

### DETAILED DESCRIPTION

Embodiments of the presently disclosed devices are described in detail with reference to the drawings, in which like reference numerals designate identical or corresponding elements in each of the several views. As used herein, the term "distal" or "leading" refers to that portion of the device that is farther from the user, while the term "proximal" or "trailing" refers to that portion of the device that is closer to the user. As used herein, the term "clinician" refers to a doctor, nurse, or other care provider and may include support personnel. As used herein, the term "subject" refers to a human patient or other animal.

Non-limiting examples of endoscopic surgical systems according to the present disclosure include manual, mechanical and/or electromechanical delivery devices such as surgical tack appliers (*e.g.,* tackers) and the like. For a more detailed description of similar endoscopic surgical systems and components thereof that can be used with, or adapted for use with, the presently described endoscopic surgical systems/devices, reference can be made to U.S. Patent Application Publication No. 2015/0005789, filed on August 23, 2013.

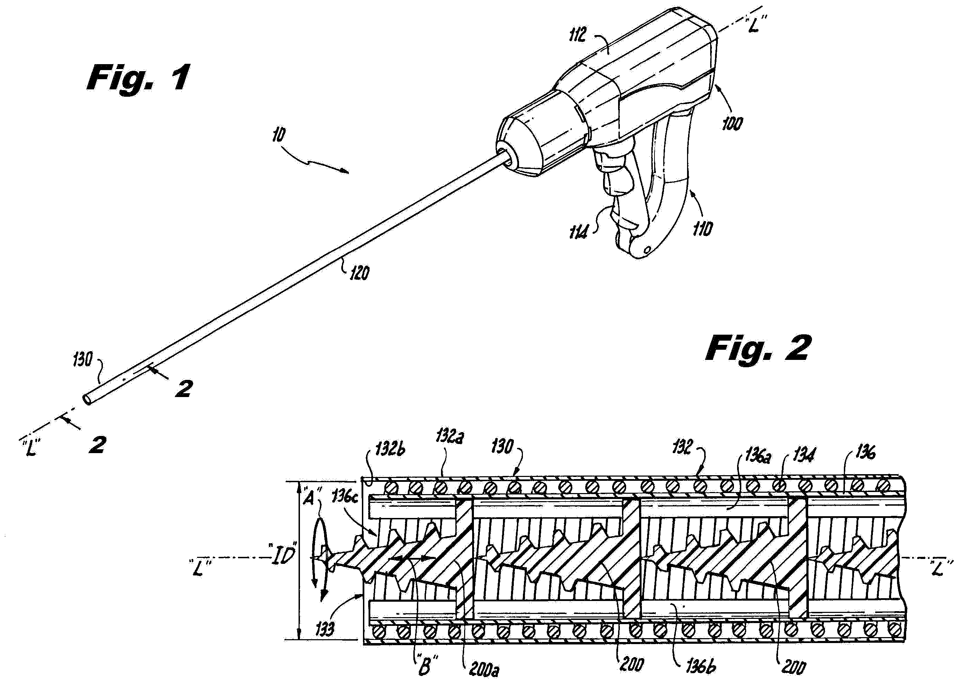

Referring initially to Fig. 1, an endoscopic surgical system is shown generally as 10. Endoscopic surgical system 10 includes an endoscopic surgical device or delivery device in the form of an endoscopic surgical tack applier or tacker 100. Tack applier 100 includes a handle assembly 110 and an elongated body portion 120 that extends distally along a longitudinal axis "L" from handle assembly 110 to a loading unit or end effector 130 at a distal end of elongated body portion 120 that is selectively detachable/attachable from/to elongated body portion 120.

Briefly, handle assembly 110 defines a longitudinal axis "L" between proximal and distal ends thereof and includes a handle housing 112, a trigger 114, and a ferrule 116 interconnecting the handle housing 112 with elongated body portion 120. Handle assembly 110 supports a drive assembly (not shown) that is operatively coupled to trigger 114.

As seen in Figs. 2-4, loading unit 130 includes an outer tube 132 that has an outer surface 132a and an inner surface 132b. Inner surface 132b defines a passage 133 and an inner diameter "ID" which may be any suitable dimension such as 5mm. Of course, the inner diameter "ID" of outer tube 132 may be smaller and/or larger than 5mm. Outer tube 132 supports a coil 134 and a drive member 136 therein. Drive member 136 includes a first tine 136a and a second tine 136b that define a channel 136c therebetween. Channel 136c of drive member 136 receives and supports fasteners 200 therein. As indicated by arrows "A," rotation of drive member 136 rotates first and second tines 136a, 136b about longitudinal axis "L" of loading unit 130. Rotation of first and second tines 136a, 136b of drive member 136 imparts rotation on fasteners 200 to advance fasteners 200 along coil 134 of loading unit 130 and axially relative to longitudinal axis "L" of loading unit 130, as indicated by arrows "B," to deploy fasteners 200 from loading unit 130 as described in greater detail below. For a more detailed description of similar tack appliers and components thereof that can be used with, or adapted for use with, the presently described tack applier, reference can be made to International Patent Application Serial Number PCT/CN2014/072951, filed on March 6, 2014.

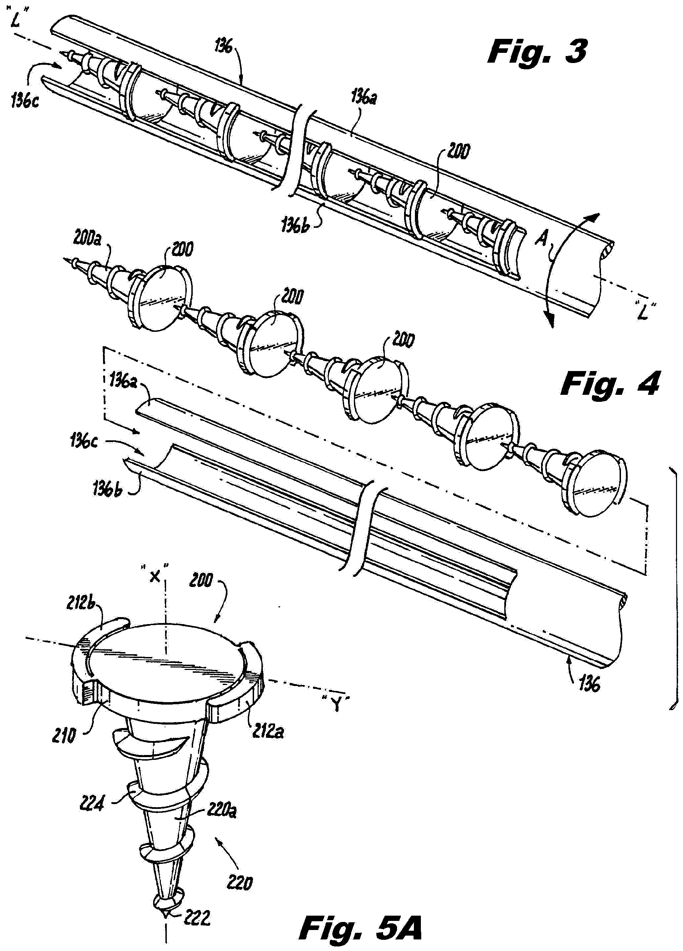

With reference to Figs. 5A-8, fastener 200 includes a head 210 and a shank 220 extending from head 210. Fastener 200 defines a central longitudinal axis "X" that extends through the head 210 and the shank 220. Central longitudinal axis "X" may be positioned in axial alignment (*e.g.,* coaxial) with longitudinal axis "L" of loading unit 130 when fastener 200 is supported within loading unit 130. Head 210 of fastener 200 includes a top surface 210a, a bottom surface 210b, and an annular side surface 210c that extends between the top and bottom surfaces 210a, 210b. Shank 220 of fastener 200 extends from bottom surface 210b of head 210 of fastener 200 to a pointed tip 222. Shank 220 is elongated and includes an outer surface 220a having one or more threads 224 extending therefrom. Threads 224 of shank 220 rotate and/or spiral around outer surface 220a of shank 220 and along at least a portion of a length of shank 220 (in the manner of a helix). In some embodiments, threads 224 are defined in outer surface 220a of shank 220.

Fastener 200 includes hinged wings 212a, 212b secured to annular side surface 210c of head 210 of fastener 200 by a respective hinge 216 of fastener 200. Wings 212a, 212b may be secured to head 210 in complementary relation to one another, *e.g.,* on opposed sides of head 210. Each hinge 216 may be a living hinge. One or more of wings 212a, 212b, head 210, and/or hinges 216 may be formed of a resilient material and/or integrally formed with one another. Each of wings 212a, 212b includes top and bottom surfaces 214a, 214b, and outer and inner side surfaces 214c, 214d that extend between top and bottom surfaces 214a, 214b of each of wings 212a, 212b. Each of wings 212a, 212b has a curved or arcuate profile that extends between a shoulder 214e at a first end thereof and a tip 214f at a second end thereof that is opposite to the first end.

Hinged wings 212a, 212b may be disposed in complementary relation to one another such that shoulder 214e of a first one of wings 212a, 212b is positioned adjacent to tip 214f of a second one of wings 212a, 212b. Wings 212a, 212b may conform to an outer surface of head 210 of fastener 200 such as annular side surface 210c of head 210. Wings 212a, 212b may extend in the same clockwise and/or counterclockwise direction about annular side surface 210c of head 210.

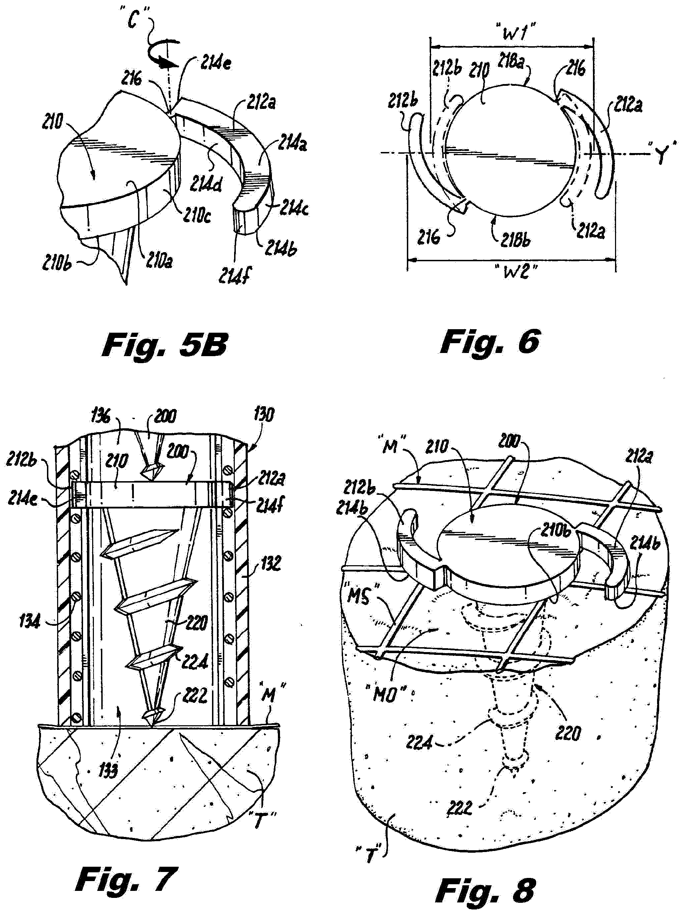

Hinges 216 enable respective hinged wings 212a, 212b to move radially outward in a lateral direction along a transverse axis "Y" defined through head 210 of fastener 200 and relative to head 210. In some embodiments, hinged wings 212a, 212b may move relative to one another and/or head 210 in the same plane. Wings 212a, 212b pivot about a respective hinge 216, as indicated by arrow "C," to move hinged wings 212a, 212b between a constrained state (Fig. 5A) and an extended state (Fig. 5B). Hinges 216 may be configured to bias hinged wings 212a, 212b toward the extended state.

As seen in Figs. 2 and 6, fastener 200 defines a first width or diameter "W1" in the constrained state and a second width or diameter "W2" in the extended state that is larger than first width or diameter "W1." First width "W1" of fastener 200 is smaller than the inner diameter "ID" of outer tube 132 of loading unit 130 to enable fastener 200 to fit within passage 133 of outer tube 132. When positioned within loading unit 130, inner surface 132b of outer tube 132 may frictionally engage and/or contact outer side surface 214c of hinged wings 212a, 212b to maintain hinged wings 212a, 212b in the constrained state. Second width "W2" of fastener 200 may be larger than inner diameter "ID" of outer tube 132 of elongated body portion 130 such that fastener 200 may bias from the constrained state to the extended state upon deployment of fastener 200 from outer tube 132 of loading unit 130, as described in greater detail below.

In the constrained state, inner side surfaces 214d of respective hinged wings 212a, 212b are in contact with annular side surface 210c of head 210 such that tine gaps 218a, 218b are defined along annular side surface 210c of head 210 and between shoulders and tips 214e, 214f of respective wings 212a, 212b. Tine gaps 218a, 218b receive respective tines 136a, 136b of drive member 136 such that shoulders 214e of respective wings 212a, 212b engage respective tines 136a, 136b of drive member 136 to enable rotation of drive member 136 to rotate wings 212a, 212b through coil 134 of loading unit 130. In the extended state, inner side surfaces 214d of respective wings 212a, 212b are spaced from annular side surface 210c of head 210.

In use, with reference to Figs. 1-8, endoscopic surgical system 10 can be used to secure, for example, a mesh "M" to tissue "T." For instance, during a hernia repair, mesh "M" is positioned on tissue "T" and a distal tip of loading unit 130 of endoscopic surgical system 10 is positioned adjacent to mesh "M." To secure mesh "M" to tissue "T," a clinician can fire loading unit 130 upon an actuation of trigger 114 of handle assembly 110. A firing of loading unit 130 rotates drive member 136 such that first and second tines 136a, 136b impart a rotational force through shoulders 214e of respective hinged wings 212a, 212b of each of fasteners 200 supported within loading unit 130. Each of fasteners 200 are supported within loading unit 130 in the constrained state. The rotational force of tines 136a, 136b rotates fasteners 200 along coil 134 of loading unit 130, as indicated by arrows "A," so that fasteners 200 translate axially relative to longitudinal axis "L," as indicated by arrows "B." To this end, each of fasteners 200 spiral along outer tube 132 of loading unit 130 such that a leading or distal-most fastener 200a deploys from loading unit 130 through a leading or distal end of outer tube 132 of loading unit 130 and into "M" and tissue "T."

As the leading fastener 200a is deployed from loading unit 130, shank 220 of fastener 200 passes through mesh "M," for example, through a mesh opening "MO" defined between mesh struts "MS," and into tissue "T" to secure mesh "M" to tissue "T." As head 210 of fastener 200a is dispensed from loading unit 130, hinged wings 212a, 212b move radially outwardly in a lateral direction from the constrained state to the extended state. Movement of hinged wings 212a, 212b from the constrained state to the extended state is a function of resiliency and/or centrifugal forces imparted to hinged wings 212a, 212b as result of the spiral/rotational movement imparted thereto from drive member 136 of loading unit 130. After being fired from the loading unit 130, at least portions of bottom surface 210b of head 210 and/or bottom surfaces 214b of hinged wings 212a, 212b contact mesh "M" to secure mesh "M" to tissue "T."

As leading fastener 200a is deployed from loading unit 130, each of the remaining fasteners 200 in loading unit 130 moves toward the distal end of loading unit 130. Trigger 114 of handle assembly 110 can then be re-actuated to re-fire the loading unit 130 as desired with each of the remaining fasteners 200 successively becoming leading fastener 200a until each of the remaining fasteners 200 are fired from loading unit 130.

Loading unit 130 and/or elongated body portion 120 may be selectively removed from handle assembly 110 and/or replaced for firing additional and/or different fasteners as desired.

Turning now to Figs. 9-12, another embodiment of a fastener, generally referred to as fastener 300, is substantially similar to fastener 200 and is only described herein to the extent necessary to describe the differences in construction and operation with respect to fastener 200. Fastener 300 includes a head 310 and a shank 220 extending from head 310. Head 310 includes a top surface 310a, a bottom surface 310b, and an annular side surface 310c extending between top and bottom surfaces 310a, 310b.

Fastener 300 includes hinged wings 312 secured to annular side surface 310c of head 310 by a respective hinge 316. Each wing 312 includes a boss 312a extending therefrom. Ledges 314 extend from annular side surface 310c of head 310 and define a respective pocket 314a therein. Pockets 314a of ledges 314 receive a boss 312a of a respective one of wings 312 to selectively constrain wings 312 against annular side surface 310c of head 310. Advantageously, this configuration of pockets 314a and bosses 312a enables fasteners 300 to maintain maximum constrained dimensions while facilitating insertion into loading unit 130 and/or advancement along loading unit 130 with minimal or no frictional resistance/contact along inner surface 132b of outer tube 132. In some embodiments, one or more hinged wings 312 and/or one or more ledges 314 include one or more bosses 312a and/or define one or more pockets 314a therein such that each pocket 314a corresponds to a boss 312a of respective wings 312 and/or ledges 314.

In use, fastener 300 is deployed from loading unit 130 similar to deployment of fastener 200 as described above with respect to fastener 200. As head 310 of fastener 300 (*e.g.,* a leading fastener 300) is dispensed from loading unit 130, centrifugal force imparted on fastener 300 from drive member 136 separates bosses and pockets 312a, 314a from respective hinged wings 312 and ledges 314. Resiliency and/or centrifugal forces applied to hinged wings 312 enable hinged wings 312 to pivot radially outwardly from head 310 in a lateral direction about hinge 316, as indicated by arrow "D," from a constrained state to an extended state.

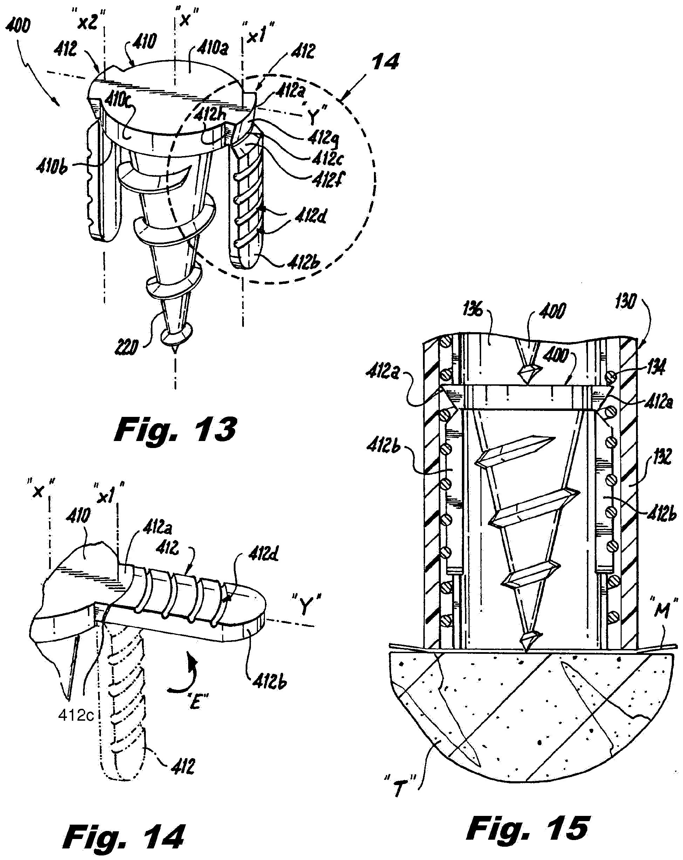

With reference to Figs. 13-17, another embodiment of a fastener, generally referred to as fastener 400, includes a head 410 and a shank 220 extending from head 410. Head 410 includes a top surface 410a, a bottom surface 410b, and an annular side surface 410c extending between top and bottom surfaces 410a, 410b. Fastener 400 includes hinged wings 412 secured to head 410 and which are movable between a constrained state (Fig. 13) and an extended state (Fig. 14). Each wing 412 includes a shoulder 412a secured to head 410, and an arm 412b secured to shoulder 412a by a hinge 412c. Arm 412b defines channels 412d in an outer surface thereof and includes a first mating surface 412f that is complementary to a second mating surface 412g of shoulder 412a. Channels 412d may define a portion of a helical thread having any suitable pitch. In embodiments, channels 412d are arcuate. First and second mating surfaces 412f, 412g are engagable to enable arm 412b to align with shoulder 412a in the extended state.

In the constrained state, arm 412b of hinged wing 412 is positioned in parallel, or substantially parallel, relation to shank 220 of fastener 400 such that longitudinal axs "X1," "X2" of hinged wings 412 are parallel to one another and a central longitudinal axis "X" of fastener 400. As illustrated in Fig. 14, arm 412b is pivotable from the constrained state to an extended state, as indicated by arrow "E." In the extended state, arm 412b extends laterally outward from annular side surface 410c of head 410 along a transverse axis "Y" through head 410 such that arm 412b is orthogonal or substantially orthogonal to central longitudinal axis "X" of fastener 400.

In use, fastener 400 is deployed from loading unit 130 similar to deployment of fasteners 200, 300 described above with respect to fasteners 200, 300. While fasteners 400 are positioned within loading unit 130 in the constrained state, channels 412d of arms 412b of fasteners 400 receive an inner surface of coil 134 of loading unit 130 and shoulders 412a are positioned between adjacent windings of coil 134. Drive member 136 engages a side surface 412h of shoulders 412a of fasteners 400 to rotate fasteners 400 along coil 134 so that fasteners axially translate along loading unit 130 for deployment from a distal end of loading unit 130.

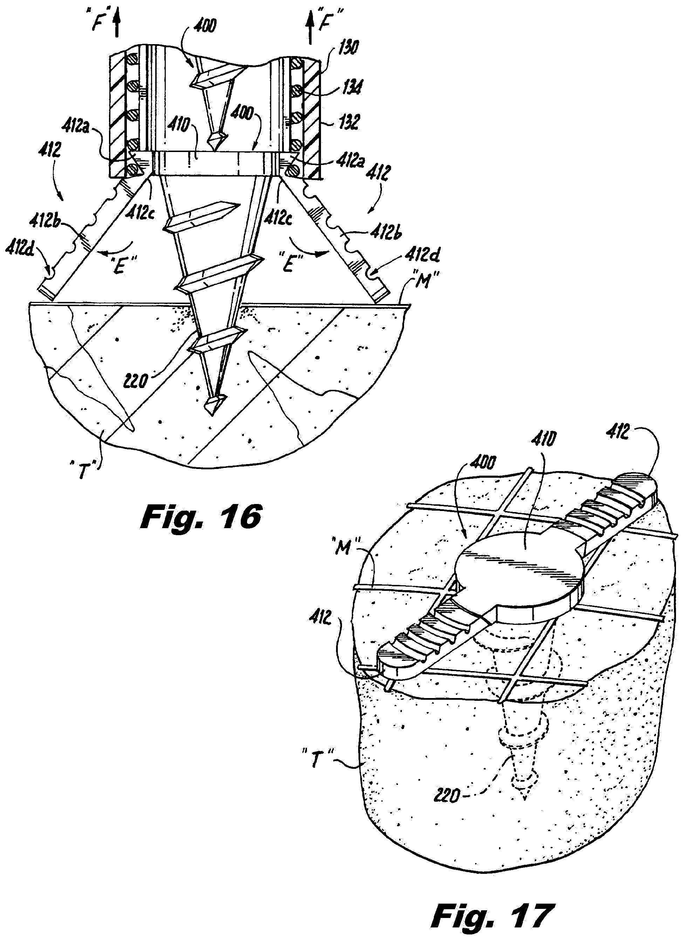

As seen in Figs. 16 and 17, shank 220 is advanced through mesh "M" and into tissue "T" such that as arms 412 of fastener 400 begin to extend through distal end of loading unit 130. With shank 220 partially embedded within tissue "T," loading unit 130 is moved proximally relative to a deploying fastener 400, as indicated by arrow "F," to impart a force on shoulders 412a of hinged wings 412 of fastener 400 through coil 134 of loading unit 130. The imparted force enables hinged arms 412 of fasteners 400 to pivot toward the extended state, as indicated by arrow "E." Centrifugal forces applied to fastener 400 via rotation of drive member 136 further facilitate movement of hinged arms 412 from the constrained state to the extended state. Similar to fasteners 200, 300, fasteners 400 can secure mesh "M" to tissue "T" with hinged arms 412 of fasteners 400 disposed in the extended state.

In some embodiments, the presently described hinged wings, or portions thereof, may include resilient members to help spring bias the hinged wings from the constrained state to the extended state. For example, the resilient members may include one or more resilient wires extending through the hinged wings. It is contemplated that any of the presently disclosed fasteners, or components thereof, can be formed, at least partially, of any suitable biodegradable and/or biocompatible material.

The various embodiments disclosed herein may also be configured to work with robotic surgical systems and what is commonly referred to as "Telesurgery." Such systems employ various robotic elements to assist the clinician and allow remote operation (or partial remote operation) of surgical instrumentation. Various robotic arms, gears, cams, pulleys, electric and mechanical motors, etc. may be employed for this purpose and may be designed with a robotic surgical system to assist the clinician during the course of an operation or treatment. Such robotic systems may include remotely steerable systems, automatically flexible surgical systems, remotely flexible surgical systems, remotely articulating surgical systems, wireless surgical systems, modular or selectively configurable remotely operated surgical systems, etc.

The robotic surgical systems may be employed with one or more consoles that are next to the operating theater or located in a remote location. In this instance, one team of clinicians may prep the subject for surgery and configure the robotic surgical system with one or more of the instruments disclosed herein while another clinician (or group of clinicians) remotely control the instruments via the robotic surgical system. As can be appreciated, a highly skilled clinician may perform multiple operations in multiple locations without leaving his/her remote console which can be both economically advantageous and a benefit to the subject or a series of subjects.

The robotic arms of the surgical system are typically coupled to a pair of master handles by a controller. The handles can be moved by the clinician to produce a corresponding movement of the working ends of any type of surgical instrument (*e.g.,* end effectors, graspers, knifes, scissors, *etc.*) which may complement the use of one or more of the embodiments described herein. The movement of the master handles may be scaled so that the working ends have a corresponding movement that is different, smaller or larger, than the movement performed by the operating hands of the clinician. The scale factor or gearing ratio may be adjustable so that the operator can control the resolution of the working ends of the surgical instrument(s).

The master handles may include various sensors to provide feedback to the clinician relating to various tissue parameters or conditions, e.g., tissue resistance due to manipulation, cutting or otherwise treating, pressure by the instrument onto the tissue, tissue temperature, tissue impedance, etc. As can be appreciated, such sensors provide the clinician with enhanced tactile feedback simulating actual operating conditions. The master handles may also include a variety of different actuators for delicate tissue manipulation or treatment further enhancing the clinician's ability to mimic actual operating conditions.

Persons skilled in the art will understand that the structures and methods specifically described herein and shown in the accompanying figures are non-limiting exemplary embodiments, and that the description, disclosure, and figures should be construed merely as exemplary of particular embodiments. It is to be understood, therefore, that the present disclosure is not limited to the precise embodiments described, and that various other changes and modifications may be effected by one skilled in the art without departing from the scope of the disclosure. Additionally, the elements and features shown or described in connection with certain embodiments may be combined with the elements and features of certain other embodiments without departing from the scope of the present disclosure, and that such modifications and variations are also included within the scope of the present disclosure.

## Claims

1. A fastener (200, 300, 400) for securement to a target tissue site, the fastener (200, 300, 400) comprising:
a head (210, 310, 410);
a shank (220) extending from the head (210, 310, 410) and including a thread (224) that extends along an outer surface (220a) of the shank (220), the thread (224) configured to secure the fastener (200, 300, 400) to the target tissue site; and
a hinged wing (212, 312, 412) secured to the head (210, 310, 410) and including a shoulder (214e), the hinged wing (212, 312, 412) movable relative to head (210, 310, 410) between a constrained state and an extended state, the shoulder (214e) engagable with a drive member (136) of a delivery device (100) while the hinged wing (212, 312, 412) is disposed in the constrained state to enable the drive member (136) to translate and rotate the fastener (200, 300, 400) through the delivery device (100) in response to movement of the drive member (136), the hinged wing (212, 312, 412) movable toward the extended state in response to deployment of the fastener (200, 300, 400) from the delivery device (100),
**characterized in that** the head (310) supports a ledge (314) that defines a pocket (314a) therein, and wherein the hinged wing (312) includes a boss (312a) extending therefrom, the pocket (314a) of the ledge (314) configured to removably receive the boss (312a) of the hinged wing (312) to maintain the hinged wing (312) in the constrained state.

2. The fastener (200, 300, 400) of claim 1, wherein the hinged wing (212, 312, 412) includes a living hinge (216, 316, 416) configured to bias the hinged wing (212, 312, 412) toward the extended state.

3. The fastener (200, 300, 400) of claim 1 or claim 2, wherein the thread (224) extends from the outer surface (220a) of the shank (220) and around the outer surface (220a) of the shank (220).

4. The fastener (200, 300, 400) of claim 3, wherein the thread (224) defines a spiral around the outer surface (220a) of the shank (220) along a length of the shank (220).

5. The fastener (200, 300) of any preceding claim, wherein the hinged wing (212, 312) has an arcuate profile that conforms to an outer surface of the head (210, 310) while the hinged wing (212, 312) is disposed in the constrained state.

6. The fastener (200, 300) of any preceding claim, wherein the hinged wing (212, 312) pivots radially outward from the head (210, 310) in a lateral direction as the hinged wing (212, 312) moves toward the extended state.

7. The fastener (200, 300, 400) of any preceding claim, further including a second hinged wing (212, 312, 412), the second hinged wing (212, 312, 412) secured to the head (210, 310, 410) and including a second shoulder (214e), the second hinged wing (212, 312, 412) movable relative to head (210, 310, 410) between a constrained state and an extended state.

8. The fastener (200, 300, 400) of claim 7, wherein the hinged wings (212, 312, 412) are supported on the head (210, 310, 410) in complementary relation to one another.

9. The fastener (200, 300, 400) of any preceding claim, wherein the fastener (200, 300, 400) is adapted to advance through a 5mm diameter passage of a delivery device (100) while the hinged wing (212, 312, 412) is disposed in the constrained state.

10. The fastener (400) of any of claims 1-4, wherein the shank (220) defines a longitudinal axis ("X"), and wherein the hinged wing (412) defines a longitudinal wing axis ("X1"), the longitudinal wing axis ("X1") disposed in parallel relation to the longitudinal axis ("X") of the shank (220) while the hinged wing (412) is disposed in the constrained state, the longitudinal wing axis ("X1") disposed in transverse relation to the longitudinal axis ("X") of the shank (220) while the hinged wing (412) is disposed in the extended state.

11. The fastener (400) of any of claims 1-4, wherein the hinged wing (412) defines a channel (412d) in an outer surface thereof, the channel (412d) defining a portion of a helical thread and adapted to be engaged by a thread (134) of a delivery device (100) to enable the fastener (400) to advance along a delivery device (100).

12. An endoscopic surgical system (10), comprising:
an end effector (130) supporting a drive member (136); and
the fastener (200, 300, 400) of any preceding claim supported in the end effector (130).

## Patentansprüche

1. Befestigungselement (200, 300, 400) für eine Anbringung an einer Zielgewebestelle, wobei das Befestigungselement (200, 300, 400) Folgendes umfasst:
einen Kopf (210, 310, 410);
einen Schaft (220), der sich aus dem Kopf (210, 310, 410) erstreckt und ein Gewinde (224) beinhaltet, das sich entlang einer Außenoberfläche (220a) des Schafts (220) erstreckt, wobei das Gewinde (224) konfiguriert ist, um das Befestigungselement (200, 300, 400) an die Zielgewebestelle anzubringen; und
einen ausklappbaren Flügel (212, 312, 412), der an dem Kopf (210, 310, 410) angebracht ist und eine Schulter (214e) beinhaltet, wobei der ausklappbare Flügel (212, 312, 412) relativ zu dem Kopf (210, 310, 410) zwischen einem eingeschränkten Zustand und einem erstreckten Zustand bewegbar ist, wobei die Schulter (214e) mit einem Antriebsbauteil (136) einer Abgabevorrichtung (100) in Eingriff nehmbar ist, während der ausklappbare Flügel (212, 312, 412) in dem eingeschränkten Zustand angeordnet ist, um es dem Antriebsbauteil (136) zu ermöglichen, das Befestigungselement (200, 300, 400) durch die Abgabevorrichtung (100) hindurch als Reaktion auf eine Bewegung des Antriebselements (136) zu verschieben und zu drehen, wobei der ausklappbare Flügel (212, 312, 412) zu dem erstreckten Zustand hin als Reaktion auf ein Lösen des Befestigungselements (200, 300, 400) aus der Abgabevorrichtung (100) bewegbar ist, **dadurch gekennzeichnet, dass** der Kopf (310) eine Verstärkungsrippe (314) stützt, die eine Tasche (314a) darin definiert, und wobei der ausklappbare Flügel (312) einen Ansatz (312a) beinhaltet, der sich daraus erstreckt, wobei die Tasche (314a) der Verstärkungsrippe (314) konfiguriert ist, um den Ansatz (312a) des ausklappbaren Flügels (312) entfernbar aufzunehmen, um den ausklappbaren Flügel (312) in dem eingeschränkten Zustand zu halten.

2. Befestigungselement (200, 300, 400) nach Anspruch 1, wobei der ausklappbare Flügel (212, 312, 412) ein Filmscharnier (216, 316, 416) beinhaltet, das konfiguriert ist, um den ausklappbaren Flügel (212, 312, 412) zu dem erstreckten Zustand hin vorzuspannen.

3. Befestigungselement (200, 300, 400) nach Anspruch 1 oder 2, wobei sich das Gewinde (224) aus der Außenoberfläche (220a) des Schafts (220) und um die Außenoberfläche (220a) des Schafts (220) herum erstreckt.

4. Befestigungselement (200, 300, 400) nach Anspruch 3, wobei das Gewinde (224) eine Spirale um die Außenoberfläche (220a) des Schafts (220) herum entlang einer Länge des Schafts (220) definiert.

5. Befestigungselement (200, 300) nach einem der vorhergehenden Ansprüche, wobei der ausklappbare Flügel (212, 312) ein bogenförmiges Profil aufweist, das einer Außenoberfläche des Kopfes (210, 310) entspricht, während der ausklappbare Flügel (212, 312) in dem eingeschränkten Zustand angeordnet ist.

6. Befestigungselement (200, 300) nach einem der vorhergehenden Ansprüche, wobei der ausklappbare Flügel (212, 312) von dem Kopf (210, 310) in einer Seitenrichtung radial nach außen schwenkt, wie sich der ausklappbare Flügel (212, 312) zu dem erstreckten Zustand hin bewegt.

7. Befestigungselement (200, 300, 400) nach einem der vorhergehenden Ansprüche, das ferner einen zweiten ausklappbaren Flügel (212, 312, 412) beinhaltet, wobei der zweite ausklappbare Flügel (212, 312, 412) an dem Kopf (210, 310, 410) angebracht ist und eine zweite Schulter (214e) beinhaltet, wobei der zweite ausklappbare Flügel (212, 312, 412) relativ zu dem Kopf (210, 310, 410) zwischen einem eingeschränkten Zustand und einem erstreckten Zustand bewegbar ist.

8. Befestigungselement (200, 300, 400) nach Anspruch 7, wobei die ausklappbaren Flügel (212, 312, 412) auf dem Kopf (210, 310, 410) in komplementärer Beziehung zueinander gestützt sind.

9. Befestigungselement (200, 300, 400) nach einem der vorhergehenden Ansprüche, wobei das Befestigungselement (200, 300, 400) angepasst ist, um durch einen Durchgang mit 5 mm Durchmesser einer Abgabevorrichtung (100) hindurch vorgeschoben zu werden, während der ausklappbare Flügel (212, 312, 412) in dem eingeschränkten Zustand angeordnet ist.

10. Befestigungselement (400) nach einem der Ansprüche 1-4, wobei der Schaft (220) eine Längsachse ("X") definiert und wobei der ausklappbare Flügel (412) eine Längsflügelachse ("X1") definiert, wobei die Längsflügelachse ("X1") parallel zu der Längsachse ("X") des Schafts (220) angeordnet ist, während der ausklappbare Flügel (412) in dem eingeschränkten Zustand angeordnet ist, wobei die Längsflügelachse ("X1") quer zu der Längsachse ("X") des Schaftes (220) angeordnet ist, während der ausklappbare Flügel (412) in dem erstreckten Zustand angeordnet ist.

11. Befestigungselement (400) nach einem der Ansprüche 1-4, wobei der ausklappbare Flügel (412) einen Kanal (412d) in einer Außenoberfläche davon definiert, wobei der Kanal (412d) einen Abschnitt eines Schraubengewindes definiert und angepasst ist, um durch ein Gewinde (134) einer Abgabevorrichtung (100) in Eingriff genommen zu werden, um es dem Befestigungselement (400) zu ermöglichen, entlang einer Abgabevorrichtung (100) vorgeschoben zu werden.

12. Endoskopisches chirurgisches System (10), das Folgendes umfasst:
einen Endeffektor (130), der ein Antriebselement (136) stützt; und
das Befestigungselement (200, 300, 400) nach einem der vorhergehenden Ansprüche, das in dem Endeffektor (130) gestützt wird.

## Revendications

1. Attache (200, 300, 400) destinée à la fixation à un site tissulaire cible, l'attache (200, 300, 400) comprenant :
une tête (210, 310, 410) ;
une tige (220) s'étendant à partir de la tête (210, 310, 410) et comportant un filetage (224) qui s'étend le long d'une surface extérieure (220a) de la tige (220), le filetage (224) étant conçu pour fixer l'attache (200, 300, 400) au site tissulaire cible ; et
une aile articulée (212, 312, 412) fixée à la tête (210, 310, 410) et comportant un épaulement (214e), l'aile articulée (212, 312, 412) étant mobile par rapport à la tête (210, 310, 410) entre un état contraint et un état étendu, l'épaulement (214e) pouvant entrer en prise avec un élément d'entraînement (136) d'un dispositif de distribution (100) tandis que l'aile articulée (212, 312, 412) est disposée dans l'état contraint pour permettre à l'élément d'entraînement (136) de translater et de mettre l'attache (200, 300, 400) en rotation à travers le dispositif de distribution (100) en réponse au mouvement de l'élément d'entraînement (136), l'aile articulée (212, 312, 412) étant mobile vers l'état d'extension en réponse au déploiement de l'attache (200, 300, 400) à partir du dispositif de distribution (100), **caractérisé en ce que**
la tête (310) supporte un rebord (314) qui définit une poche (314a) en son sein, et l'aile articulée (312) comportant un bossage (312a) s'étendant à partir de celui-ci, la poche (314a) du rebord (314) étant conçue pour recevoir de manière amovible le bossage (312a) de l'aile articulée (312) afin de maintenir l'aile articulée (312) dans l'état contraint.

2. Attache (200, 300, 400) selon la revendication 1, l'aile articulée (212, 312, 412) comportant une charnière vivante (216, 316, 416) conçue pour solliciter l'aile articulée (212, 312, 412) vers l'état étendu.

3. Attache (200, 300, 400) selon la revendication 1 ou la revendication 2, le filetage (224) s'étendant à partir de la surface extérieure (220a) de la tige (220) et autour de la surface extérieure (220a) de la tige (220).

4. Attache (200, 300, 400) selon la revendication 3, le filetage (224) définissant une spirale autour de la surface extérieure (220a) de la tige (220) sur une longueur de la tige (220).

5. Attache (200, 300) selon l'une quelconque des revendications précédentes, l'aile articulée (212, 312) ayant un profil arqué qui épouse une surface extérieure de la tête (210, 310) tandis que l'aile articulée (212, 312) est disposée dans l'état contraint.

6. Attache (200, 300) selon l'une quelconque des revendications précédentes, l'aile articulée (212, 312) pivotant radialement vers l'extérieur à partir de la tête (210, 310) dans une direction latérale lorsque l'aile articulée (212, 312) se déplace vers l'état étendu.

7. Attache (200, 300, 400) selon l'une quelconque des revendications précédentes, comportant en outre une seconde aile articulée (212, 312, 412), la seconde aile articulée (212, 312, 412) étant fixée à la tête (210, 310, 410) et comportant un second épaulement (214e), la seconde aile articulée (212, 312, 412) étant mobile par rapport à la tête (210, 310, 410) entre un état contraint et un état étendu.

8. Attache (200, 300, 400) selon la revendication 7, les ailes articulées (212, 312, 412) étant supportées sur la tête (210, 310, 410) en relation complémentaire l'une avec l'autre.

9. Attache (200, 300, 400) selon l'une quelconque des revendications précédentes, l'attache (200, 300, 400) étant adaptée pour avancer à travers un passage de 5 mm de diamètre d'un dispositif de distribution (100) tandis que l'aile articulée (212, 312, 412) est disposée dans l'état contraint.

10. Attache (400) selon l'une quelconque des revendications 1 à 4, la tige (220) définissant un axe longitudinal (« X »), et l'aile articulée (412) définissant un axe longitudinal de l'aile (« X1 »), l'axe longitudinal de l'aile (« X1 ») étant disposé parallèlement à l'axe longitudinal (« X ») de la tige (220) tandis que l'aile articulée (412) est disposée dans l'état contraint, l'axe longitudinal de l'aile (« X1 ») étant disposé en relation transversale à l'axe longitudinal (« X ») de la tige (220) tandis que l'aile articulée (412) est disposée à l'état déployé.

11. Attache (400) selon l'une quelconque des revendications 1 à 4, l'aile articulée (412) définissant un canal (412d) dans une surface extérieure de celle-ci, le canal (412d) définissant une partie d'un filetage hélicoïdal et étant adapté pour être mis en prise par un filetage (134) d'un dispositif de distribution (100) pour permettre à l'attache (400) d'avancer le long d'un dispositif de distribution (100).

12. Système chirurgical endoscopique (10), comprenant :
un effecteur (130) supportant un élément d'entraînement (136) ; et
l'attache (200, 300, 400) selon l'une quelconque des revendications précédentes supportée dans l'effecteur (130).
